# EUROPEAN PATENT SPECIFICATION

(11) **EP 3 998 091 B1**
(45) Date of publication and mention of the grant of the patent: **04.06.2025**
(21) Application number: 20902407.4
(22) Date of filing: 21.04.2020
(51) Int. Cl.: A61L 27/50, A61L 27/26, C12N 5/0793, A61L 27/58, C12N 5/00

(54) **TISSUE ENGINEERING MATERIAL FOR NERVE INJURY REPAIR, PREPARATION METHOD THEREFOR AND APPLICATION THEREOF**
GEWEBEZÜCHTUNGSMATERIAL ZUR REPARATUR VON NERVENLÄSIONEN, VERFAHREN ZU SEINER HERSTELLUNG UND SEINE VERWENDUNG
MATÉRIAU D'INGÉNIERIE TISSULAIRE POUR LA RÉPARATION DE LÉSIONS NERVEUSES, PROCÉDÉ DE PRÉPARATION ASSOCIÉ ET APPLICATION CORRESPONDANTE

(30) Priority: 18.12.2019 CN 201911312837
(43) Date of publication of application: 18.05.2022
(73) Proprietor: Institute Of Genetics And Developmental Biology, Chinese Academy Of Sciences, Beijing 100101 (CN)
(72) Inventor: DAI, Jianwu, Beijing 100101 (CN); LIU, Weiyuan, Beijing 100101 (CN); ZHAO, Yannan, Beijing 100101 (CN); CHEN, Bing, Beijing 100101 (CN); XIAO, Zhifeng, Beijing 100101 (CN)
(74) Representative: Nederlandsch Octrooibureau
(86) International application number: PCT/CN2020/085855
(87) International publication number: WO 2021/120471

(56) References cited:
- CN-A- 101 979 106
- CN-A- 109 010 915
- CN-A- 109 771 699
- CN-B- 101 979 106
- US-A1- 2014 113 372
- VEGA L. JOHANA C. M. ET AL: "Three dimensional conjugation of recombinant N-cadherin to a hydrogel for in vitro anisotropic neural growth", JOURNAL OF MATERIALS CHEMISTRY. B, vol. 4, no. 42, 1 January 2016 (2016-01-01), GB, pages 6803 - 6811, XP055948909, ISSN: 2050-750X, Retrieved from the Internet <URL:https://pubs.rsc.org/en/content/articlepdf/2016/tb/c6tb01814a> [retrieved on 20220804], DOI: 10.1039/C6TB01814A
- JINNOU HIDEO ET AL: "Radial Glial Fibers Promote Neuronal Migration and Functional Recovery after Neonatal Brain Injury", CELL STEM CELL, ELSEVIER, CELL PRESS, AMSTERDAM, NL, vol. 22, no. 1, 21 December 2017 (2017-12-21), pages 128, XP085331007, ISSN: 1934-5909, DOI: 10.1016/J.STEM.2017.11.005
- JINNOU HIDEO; SAWADA MASATO; KAWASE KOYA; KANEKO NAOKO; HERRANZ-PÉREZ VICENTE; MIYAMOTO TAKUYA; KAWAUE TAKUMI; MIYATA TAKAKI; TABA: "Radial Glial Fibers Promote Neuronal Migration and Functional Recovery after Neonatal Brain Injury", CELL STEM CELL, ELSEVIER, CELL PRESS, AMSTERDAM, NL, vol. 22, no. 1, 21 December 2017 (2017-12-21), AMSTERDAM, NL, pages 128, XP085331007, ISSN: 1934-5909, DOI: 10.1016/j.stem.2017.11.005

## Description

### Technical Field

The present invention relates to the technical field of tissue engineering, specifically, to a N-cadherin crosslinked biodegradable material for nerve injury repair and a preparation method therefor and application thereof.

### Background Art

Spinal cord, as an important part of the central nervous system, is the conduction pathway between the brain and peripheral nervous system. Spinal cord injury can lead to complete or partial loss of motor and sensory functions below the injured section of patients, which greatly reduces the quality of life of the patients and brings a huge burden to the society. At present, the clinical treatments for spinal cord injury are mainly medication such as neurotrophins and surgical decompression, but these means cannot regenerate the injured spinal cord, and thus cannot improve their motor functions, either.

With the development of tissue engineering, scaffold material combined with stem cells has brought new hope for the treatment of spinal cord injury. Studies have shown that 5 days after spinal cord injury in rats, Nestin⁺ neural stem cells will be activated in the intact segments adjacent to the injury region and spontaneously migrate to the injury region, but such migration is spatially limited, and it is difficult for neural stem cells to migrate to the central part of the injury region, so it is difficult to provide sufficient seed cells for tissue regeneration. At the same time, due to the existence of inhibitory factors such as myelin protein and chondroitin sulfate proteoglycan, the neural stem cells in the injury region tend to differentiate into glial cells rather than neurons.

Tissue engineering scaffolds are generally considered to have the characteristics of good biocompatibility. The spinal cord is composed of bundles of nerve fibers, so the scaffold material should also have the function of guiding the ordered growth of new neurons in the longitudinal direction. Collagen, as a kind of animal-derived extracellular matrix protein, has the advantages of biocompatibility and degradability, but the mechanical strength of which is poor, greatly limiting the application thereof. In addition, the scaffold materials used for the treatment of spinal cord injury should also maintain a stable morphology without bending when being immersed in cerebrospinal fluid, so as to provide a stable mechanical environment for the migration, differentiation and maturation of seed cells. CN 101979106 B discloses a tissue engineering material comprising a 151 IgG protein covalently linked to a collagen scaffold in combination with BDNF for repairing spinal cord injury. Vega *et al.* discloses a three-dimensional alginate hydrogel matrix comprising recombinant N-cadherin which promotes neuronal cell attachment and formation of a neural network. (Vega et al. Three Dimensional Conjugation of Recombinant N-Cadherin to a Hydrogel for In Vitro Anisotropic Neural Growth. J Mater Chem B. 2016;4(42):6803-6811). At present, the facilitation role of tissue-engineered scaffolds for spinal cord injury repair in the migration of neural stem cells and neuronal differentiation still needs to be improved. Therefore, the development of tissue-engineered scaffolds that can efficiently promote the migration of neural stem cells and neuronal differentiation is of great significance for the repair of spinal cord injury and the recovery of motor functions.

### Summary of the Invention

In order to solve the technical problems in the prior art, the object of the present invention is to provide a tissue engineering material capable of effectively promoting the migration of neural stem cells and their differentiation into neurons, the tissue engineering material can be used for nerve injury repair; another object of the present invention is to provide a method for preparing a tissue engineering material capable of effectively promoting migration of neural stem cells and their differentiation into neurons.

In order to achieve the above objects, the present invention has carried out the screening of substances capable of effectively inducing the migration of neural stem cells when being used as tissue engineering materials, as well as the matching screening of suitable scaffold materials thereof. Through a large number of researches, the present invention has discovered that, the neural cadherin (N-cadherin) can not only form homotypic binding and mediate cell-to-cell adhesion through an extracellular domain, but can also efficiently induce the migration of the neural stem cells, enrich the neural stem cells and effectively promote the differentiation of the neural stem cells into neurons through the binding to the N-cadherin expressed by the neural stem cells when N-cadherin is crosslinked to the surface of the tissue engineering scaffolds, which is different from many factors that can only play efficient roles between cells; at the same time, the crosslinking treatment can also significantly improve the mechanical properties of the material.

Specifically, the technical solutions of the present invention are as follows:
In a first aspect, the present invention provides a tissue engineering material for nerve injury repair, which is a N-cadherin covalently crosslinked biodegradable material; the biodegradable material is collagen.

Among many biodegradable materials, the present invention finds that the collagen material can better crosslinked with the N-cadherin, the structure and the mechanical properties of the collagen material after crosslinking treatment are remarkably improved while ensuring the amount of N-cadherin crosslinked, and a more stable mechanical environment is provided for the migration of neural stem cells.

Therefore, the biodegradable material of the present invention is collagen, more preferably, is a linear ordered collagen scaffold (LOCS).

Considering comprehensively the crosslinking efficiency of the linear ordered collagen scaffold and the action effect of the N-cadherin, the linear ordered collagen scaffold and N-cadherin are preferably crosslinked in the following ratio: one linear ordered collagen scaffold having a diameter of 0.25mm and a length of 4cm: (1-6) µg of N-cadherin. More preferably, they are crosslinked in the following ratio: one linear ordered collagen scaffold having a diameter of 0.25mm and a length of 4 cm: (2.5-5) µg of N-cadherin.

According to the invention, N-cadherin is covalently crosslinked to the linear ordered collagen scaffold.

More preferably, the crosslinking agents used for covalent crosslinking are Traut's reagent and Sulfo-SMCC.

Using the above crosslinking agent, N-cadherin can be covalently crosslinked to the linear ordered collagen scaffold by crosslinking agents Sulfo-SMCC and Traut's reagent in sequence.

In a second aspect, the present invention provides a preparation method for a tissue engineering material for nerve injury repair, comprising: crosslink N-cadherin with a biodegradable material covalently to obtain a N-cadherin crosslinked biodegradable material; that biodegradable material is collagen; more preferably is a linear ordered collagen scaffold.

Preferably, a ratio of the linear ordered collagen scaffold to N-cadherin is one linear ordered collagen scaffold having a diameter of 0.25mm and a length of 4cm: (1-6) µg of N-cadherin.

More preferably, the ratio of the linear ordered collagen scaffold to N-cadherin is one linear ordered collagen scaffold having a diameter of 0.25mm and a length of 4cm: (2.5-5) µg of the N-cadherin.

Specifically, the preparation method of the tissue engineering material for nerve injury repair comprises the following steps:
(1) treating a linear ordered collagen scaffold with Traut's reagent to obtain a linear ordered collagen scaffold modified by Traut's reagent; and
(2) treating the linear ordered collagen scaffold modified by Traut's reagent with Sulfo-SMCC and N-cadherin.

The present invention has found that the linear ordered collagen scaffold modified by Traut's reagent can covalently bind to N-cadherin modified by Sulfo-SMCC more efficiently to improve the crosslinking efficiency, and the obtained linear ordered collagen scaffold crosslinked with N-cadherin has a stable and ordered topological structure and mechanical properties.

Preferably, the ratio of the linear ordered collagen scaffold to the Traut's reagent is one linear ordered collagen scaffold having a diameter of 0.25mm and a length of 4cm: (0.05-0.15) mg of Traut's reagent. The molar ratio of the N-cadherin to the Sulfo-SMCC is 1: (60-100).

In the above step (1), the concentration of the Traut's reagent is preferably 0.2mg/mL to 5mg/ml.

In the above step (2), the concentration of the N-cadherin is preferably 200µg/mL to 400µg/mL. The concentration of the Sulfo-SMCC is preferably 0.3mg/mL to 3.5mg/mL.

In the above step (1), the treatment time is preferably 1h to 3h.

In the above step (2), the treatment time is preferably 1h to 3h.

Both step (1) and the step (2) are preferably carried out at a temperature of 18°C to 25°C.

In a third aspect, the present invention provides the use of the tissue engineering material for nerve injury repair in preparing a material for nerve injury repair.

Preferably, that nerve injury is a spinal cord nerve injury.

In a fourth aspect, the present invention provides the tissue engineering material for use in nerve injury repair in promoting directed migration of neural stem cells or promoting differentiation of neural stem cells.

The advantageous effect of the present invention lies in that the tissue engineering material (LOCS-Ncad) prepared by crosslinking N-cadherin with the linear ordered collagen scaffold can efficiently induce migration of neural stem cells towards an injury region (especially a central region of the injury region), enrich the neural stem cells in the injury region, and effectively inhibit the deposition of inhibitory factors such as chondroitin sulfate proteoglycan and the like, promote differentiation of the neural stem cells into neurons, and further promote the recovery of motor functions; at the same time, the obtained N-cadherin crosslinked linear ordered collagen scaffold also has a stable and ordered topology structure and excellent mechanical properties (the tensile modulus is remarkably improved, and the linear state is stable), so it can be used to repair nerve injuries such as spinal cord injury and the like.

### Brief Description of the Drawings

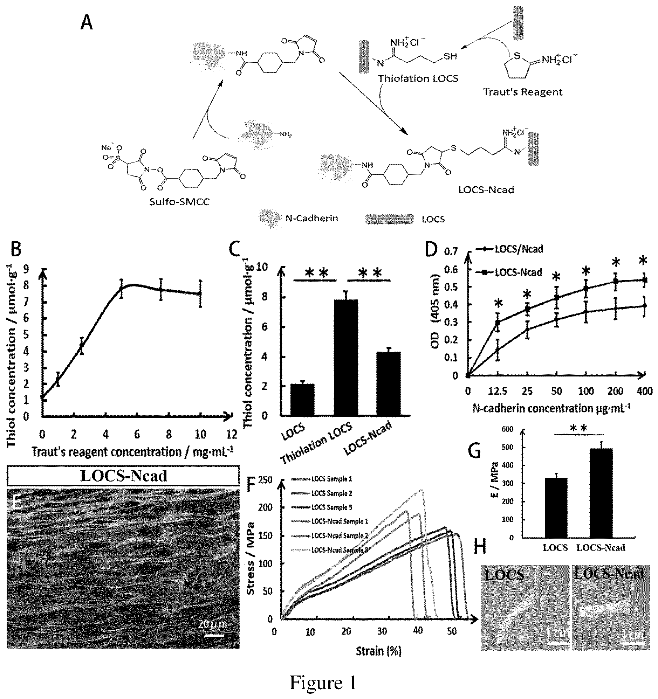

Fig. 1 shows the preparation and performance characterization of LOCS-Ncad in Experimental Examples 1 and 2 of the present invention. (A) shows a schematic diagram of two-step crosslinking method for the preparation of LOCS-Ncad; (B) shows the concentration of sulfhydryl groups after treatment with different concentration of Traut's reagent detected by Ellman's reaction; (C) shows the concentration of sulfhydryl groups in untreated LOCS (LOCS), LOCS after treatment with 5mg/mL of Traut's reagent (Thiolation LOCS), and after further reaction with Sulfo-SMCC/N-cadherin (LOCS-Ncad); (D) shows the binding amounts of N-cadherin of crosslinking treatment (LOCS-Ncad) and natural adsorption (LOCS/Ncad) detected by ELISA; (E) shows the linear ordered structure of LOCS-Ncad confirmed by scanning electron microscopy; (F) shows stress-strain curves of LOCS-Ncad and LOCS; (G) shows Young's modulus of LOCS-Ncad and LOCS; and (H) shows morphology of LOCS-Ncad and LOCS after immersion in normal saline for 30min; * represents p < 0.05, and ** represents p < 0.01.

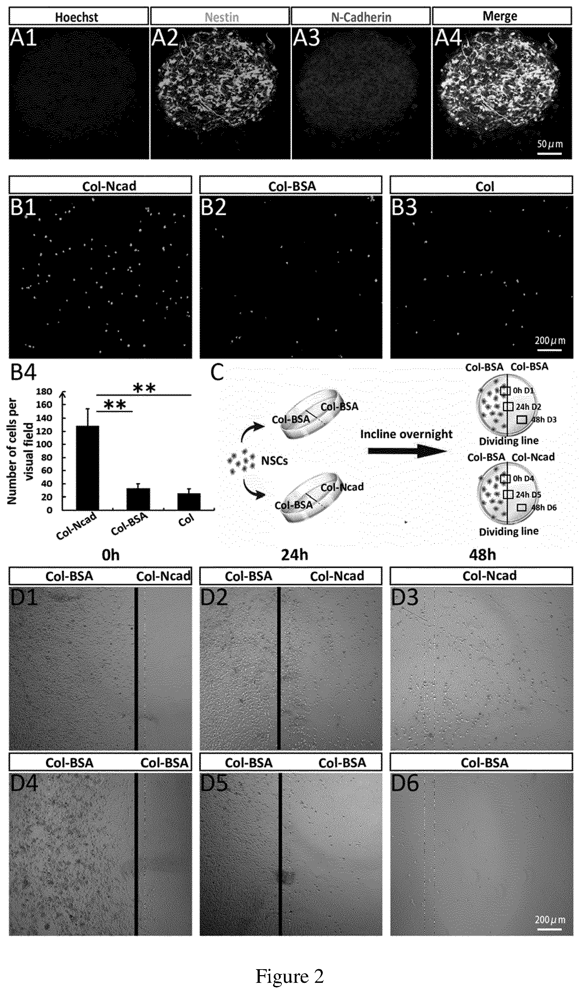

Fig. 2 shows that a two-dimensional simulation material of LOCS-Ncad promotes the adhesion and migration of neural stem cells in Experimental Example 3 of the present invention. (A1) to (A4) show that immunofluorescence staining confirms the expression of N-cadherin in neural stem cells, Hoechst represents Hoechst staining, Nestin represents Nestin protein, and N-cadherin represents neural cadherin; (B1) to (B4) show the adhesion of neural stem cells on different modified surfaces, Col-Ncad represents a two-dimensional simulation material of LOCS-Ncad, Col-BSA represents a two-dimensional simulation material of LOCS-BSA, and Col represents a collagen gel; (C) shows schematic diagram of migration experiment design; and (D1) to (D6) show migration experiment results; * represents p < 0.05, and ** represents p < 0.01.

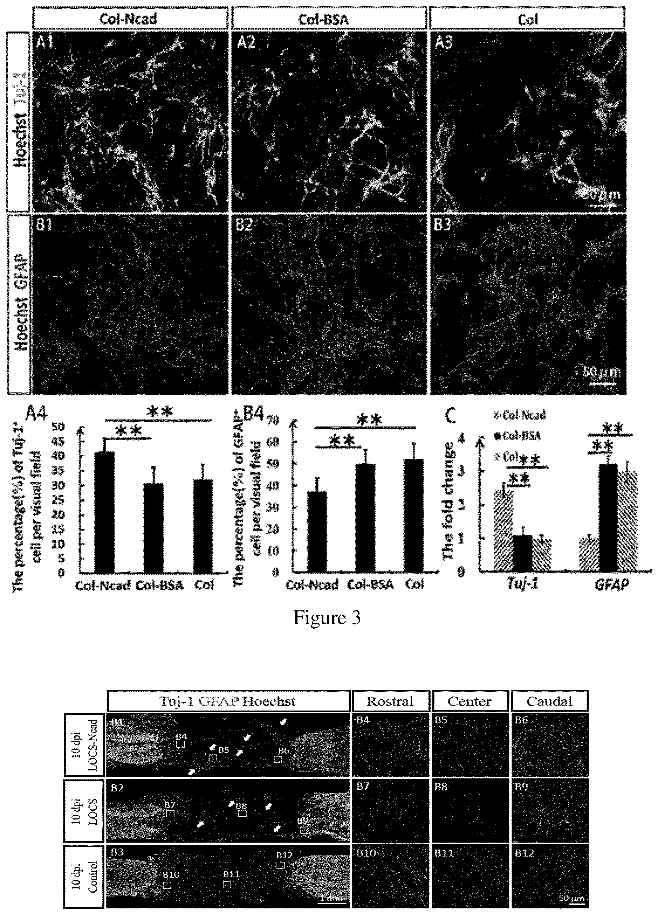

Fig. 3 shows that a two-dimensional simulation material of LOCS-Ncad promotes differentiation of neural stem cells into neurons in Experimental Example 3 of the present invention. (A1) to (A4) show the proportion of Tuj-1 (a neuron marker) positive cells determined by immunofluorescence staining after neural stem cells differentiated on the surfaces of two-dimensional simulation material of LOCS-Ncad (Col-Ncad), two-dimensional simulation material of LOCS-BSA (Col-BSA) and two-dimensional simulation material of LOCS (Col) for 7 days, respectively; (B1) to (B4) show the proportion of GFAP (an astrocyte marker) positive cells determined by immunofluorescence staining after neural stem cells differentiate on the surface of two-dimensional simulation material of LOCS-Ncad(Col-Ncad), two-dimensional simulation material of LOCS-BSA (Col-BSA) and two-dimensional simulation material of LOCS (Col) for 7 days, respectively; and (C) shows changes in expression of Tuj-1 and GFAP in neural stem cells on the surfaces of two-dimensional simulation material of LOCS-Ncad (Col-Ncad), two-dimensional simulation material of LOCS-BSA (Col-BSA) and two-dimensional simulation material of LOCS (Col) detected by qPCR; * represents p < 0.05, and ** represents p < 0.01.

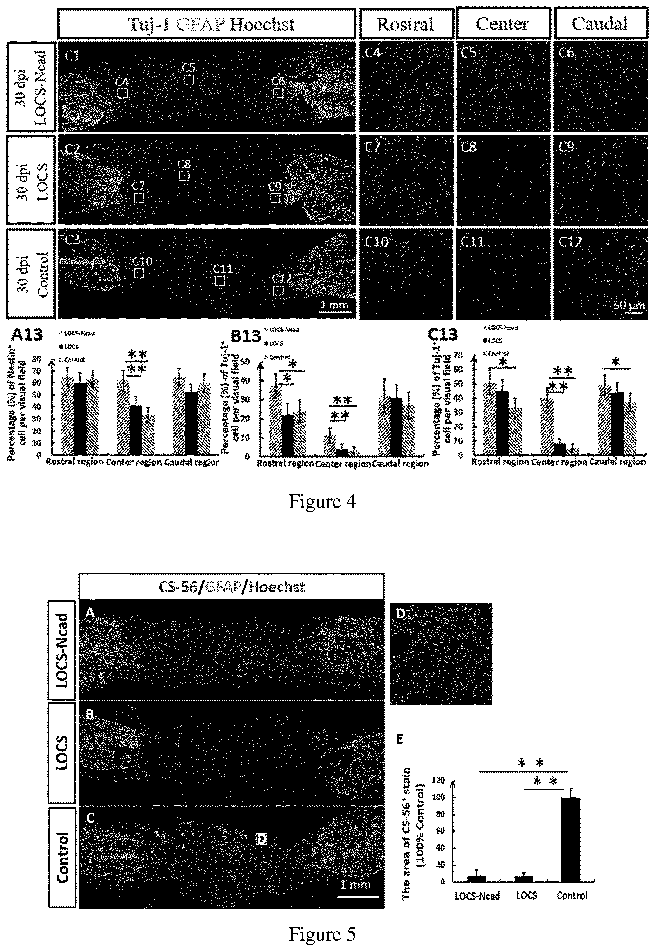

Fig. 4 shows that LOCS-Ncad promotes the migration of endogenous neural stem cells to the center of an injury region in a spinal cord injury model in Experimental Example 4 of the present invention. (A1) to (A13) show migration of endogenous neural stem cells on LOCS-Ncad and LOCS scaffolds after 10 days of complete spinal cord transection in rats; (B1) to (B13) show the proportion of endogenous neural stem cells differentiated into neuron cells on different scaffolds after 10 days of complete spinal cord transection in rats; (C1) to (C13) show the proportion of endogenous neural stem cells differentiated into neuron cells on LOCS-Ncad and LOCS scaffolds after 30 days of complete spinal cord transection in rats; Control represents the group without any transplantation treatment; Rostral represents the headend portion of the graft material, Center represents the central portion of the graft material, and Caudal represents the caudal portion of the graft material. * represents p < 0.05, and ** represents p < 0.01.
Fig. 5 shows that LOCS-Ncad inhibits the deposition of chondroitin sulfate proteoglycans (CSPGs) in the spinal cord injury model in Experimental Example 4 of the present invention; wherein A, B and C show the CSPGs deposition on LOCS-Ncad, LOCS and the control group after 30 days of complete spinal cord transection in rats, respectively; D shows an enlarged detail view of CSPGs deposition in the control group; and E shows the statistical result of the CSPGs deposition.

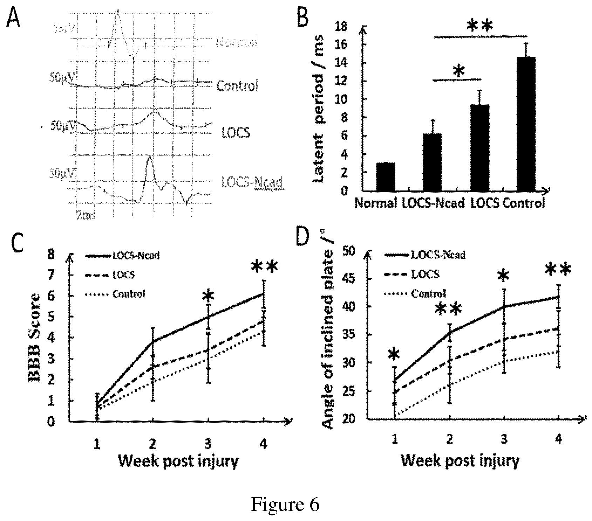

Fig. 6 shows that the transplantation of LOCS-Ncad promotes electrophysiological and motor functions recovery in rats with complete transection spinal cord injury in Experimental Example 5 of the present invention. (A) shows the electrophysiological representative curves of LOCS-Ncad and LOCS scaffold transplantation groups, control group (Control) without any transplantation treatment, and normal group (Normal) 30 days after complete spinal cord transection in rats; (B) shows the changes in the latency after the transplantation of LOCS-Ncad and LOCS scaffold 30 days after complete spinal cord transection in rats; (C) shows the BBB score results of rats after the transplantation of LOCS-Ncad and LOCS scaffolds 30 days after complete spinal cord transection; and (D) shows the results of the oblique plate test in rats after the transplantation of LOCS-Ncad and LOCS scaffolds 30 days after complete spinal cord transection,; * represents p < 0.05, and ** represents p < 0.01.

### Specific Modes for Carrying Out the Embodiments

Preferred embodiments of the present invention will be described in detail below with reference to Examples. It is to be understood that the following Examples are given for illustrative purposes only and are not intended to limit the scope of the invention which is defined in the appended claims. Various modifications and substitutions can be made to the present invention by a person skilled in the art without departing from the scope of the present invention as defined in the appended claims.

Unless otherwise specified, the experimental methods used in the following Examples are conventional methods.

Unless otherwise specified, the materials, reagents and the like used in the following examples are commercially available, wherein Traut's reagent was purchased from Sigma corporation under Art. No. I6256; N-cadherin recombinant protein was purchased from R&D Co., Ltd., under Art. No. 6626-NC-050; and Sulfo-SMCC was purchased from Sigma corporation under Art. No. M6035.

Any references in the description to methods of treatment refer to the material of the present invention for use in a method for treatment of the human (or animal) body by therapy.

In the following Examples, the linear ordered collagen scaffold was prepared as follows:
(1) the isolated bovine fascia tissue was taken, and muscle and fat were removed;
(2) the resultant was immersed in acetone, stood at 16°C for 24 hours, and then placed in deionized water for immersion cleaning for three times (2 hours each time);
(3) the resultant was immersed in 0.2% by mass sodium lauryl sulfate aqueous solution, stood at 16°C for 24 hours, and then placed in deionized water for immersion cleaning for 3 times (2 hours each time);
(4) the resultant was immersed in 1% by mass TritonX-100 aqueous solution, stood at 16°C for 24 hours, and then placed in deionized water for immersion cleaning for 3 times (2 hours each time);
(5) the resultant was immersed in 0.25% by mass trypsin aqueous solution, stood at 37°C for 30 minutes, and then placed in deionized water for immersion cleaning for 3 times (2 hours each time);
(6) the resultant was subjected to freeze-drying (at a vacuum degree of 0.1Mpa for 48 hours) to obtain a collagen scaffold.

### Example 1: Preparation of N-cadherin crosslinked linear ordered collagen scaffolds (1)

The present invention provides an N-cadherin crosslinked LOCS (LOCS-Ncad), which is obtained by crosslinking N-cadherin to LOCS by using crosslinking agents Traut's and Sulfo-SMCC.

The preparation method of the above-mentioned N-cadherin crosslinked LOCS was specifically as follows (a schematic diagram of the reaction principle of crosslinking N-cadherin on a linear ordered collagen scaffold is shown in Fig. 1A):
(1) a bunch of LOCS (the size of a bunch of LOCS is 4cm in length and 4mm in diameter, and the size of each LOCS is 4cm in length and 0.25mm in diameter) was immersed in 300µL of Traut's reagent (PBS, pH=8, 3mM EDTA) with a concentration of 5mg/mL at room temperature for 2h; after immersing, the resultant was subjected to immersion cleaning with PBS for 3 times, 5 minutes each time, to obtain a LOCS modified by Traut's reagent; and
(2) the LOCS modified by Traut's reagent obtained in step (1) was immersed in 200µL of PBS solution (PBS, pH=7.2, 3mM EDTA) containing N-cadherin recombinant protein and Sulfo-SMCC (in which the final concentration of the N-cadherin recombinant protein was 200µg/mL, and the final concentration of Sulfo-SMCC was 1.6mg/mL) for 2h at room temperature to obtain an N-cadherin crosslinked LOCS.

### Example 2: Preparation of N-cadherin crosslinked linear ordered collagen scaffolds (2)

(1) a bunch of LOCS (the size of a bunch of LOCS is 4cm in length and 4mm in diameter, and the size of each LOCS is 4cm in length and 0.25mm in diameter) was immersed in 300µL of Traut's reagent (PBS, pH=8, 3mM EDTA) with a concentration of 5mg/mL at room temperature for 2h; after immersing, the resultant was subjected to immersion cleaning with PBS for 3 times, 5 minutes each time, to obtain a LOCS modified by Traut's reagent; and
(2) the LOCS modified by Traut's reagent obtained in step (1) was immersed in 200µL of PBS solution (PBS, pH=7.2, 3mM EDTA) containing N-cadherin recombinant protein and Sulfo-SMCC (in which the final concentration of the N-cadherin recombinant protein was 400µg/mL, and the final concentration of Sulfo-SMCC was 3.2mg/mL) for 2h at room temperature to obtain an N-cadherin crosslinked LOCS.

### Comparative Example 1: Preparation of BSA crosslinked linear ordered collagen scaffold

The present Comparative Example provides a BSA (bovine serum albumin) crosslinked LOCS, and the present Comparative Example is the same as Example 1 expected that the N-cadherin recombinant protein was replaced with BSA.

### Experimental Example 1: Effect of amount of Traut's reagent and N-cadherin recombinant protein on crosslinking efficiency

Based on the principle of crosslinking N-cadherin to LOCS by crosslinking agents Traut's reagent and Sulfo-SMCC, the crosslinking of N-cadherin was analyzed through the detection of sulfhydryl group concentration, which is specified as follows:
In the preparation process of the N-cadherin crosslinked linear ordered collagen scaffold, a bundle of LOCS (the size of which is 4cm in length and 4mm in diameter) was immersed in 300µL of Traut's reagent (PBS, pH=8, 3mM EDTA) with a concentration of 0, 1, 2.5, 5, 7.5 and 10 mg/mL at room temperature for 2h respectively, to obtain a LOCS diluted with Traut's reagent. The concentration of sulfhydryl group after treatment with different concentration of Traut's reagent were detected, and the results are shown in Figure 1B. The results show that the concentration of sulfhydryl group in LOCS (Example 1 and Example 2) treated with 5 mg/mL of Traut's reagent reaches the highest.

At the same time, the concentration of sulfhydryl group after treatment with Sulfo-SMCC and N-cadherin was detected, and the results are shown in Fig. 1C. The results show that compared with LOCS modified by Traut's reagent, the concentration of sulfhydryl group of N-cadherin crosslinked LOCS is significantly reduced, demonstrating the effectiveness of the crosslinking method.

The crosslinking amounts of N-cadherin when different amounts of N-cadherin were added (a volume of 200 µL, and concentrations of 12.5, 25, 50, 100, 200 and 400µg/mL, respectively) were detected by ELISA method, and the natural adsorption group with no cross linking agent added and only LOCS and N-cadherin was used as a control, the results are shown as D in Fig. 1. The results showed that the crosslinking amount of N-cadherin reached the highest when 200µg/mL of N-cadherin was added, and it was significantly higher than that in the natural adsorption group. It further demonstrates the effectiveness of the crosslinking methods provided in Examples 1 and 2.

### Experimental Example 2: Performance test of LOCS-Ncad

The structure of LOCS-Ncad prepared in Example 1 was detected by a scanning electron microscope, the result is shown in E of Fig. 1, and the ordered topology structure of LOCS-Ncad can be seen.

Further, the mechanical properties of LOCS-Ncad prepared in Example 1 were tested, and the stress-strain curves of which were shown in F of Fig. 1 and the Young's modulus was shown in G of Fig. 1. The results showed that compared with LOCS that no N-cadherin is crosslinked, the tensile modulus of LOCS-Ncad was significantly improved. The LOCS-Ncad was immersed in normal saline for 30min to observe its morphological changes, and the results are shown as H in Fig. 1, after being immersed in normal saline for 30min, LOCS-Ncad still maintained a linear state without bending.

### Experimental Example 3: Adhesion, migration and differentiation into neurons of neural stem cells in vitro promoted by LOCS-Ncad

The rat neural stem cells were cultured in three-dimensional culture method *in vitro,* and the results of the immunofluorescence experiment showed that the neural stem cells highly expressed N-cadherin, which provided a basis for the adhesion of LOCS-Ncad to the neural stem cells (A1 to A4 in Fig. 2).

In order to facilitate clear imaging and prove that the collagen materials modified with N-cadherin could increase adhesion to neural stem cells and promote their migration and differentiation into neurons at the same time, a two-dimensional simulation material (Col-Ncad) of LOCS-Ncad was prepared, and the preparation method is as follows: the rat tail collagen tissues of adult rats were peeled off, and dissolved with 0.5 M acetic acid for 72h, the resultant was centrifuged to obtain the supernatant, a 48-well plate was coated with the obtained supernatant at 200 µL per well, after overnight at 4°C, the supernatant was aspirated and air-drying was performed (a Col obtained after air-drying was used as a control of the two-dimensional simulation material without crosslinking any substance). After air-drying, two-step crosslinking treatment was continuously performed using the preparation method of the crosslinked N-cadherin linear ordered collagen scaffold of Example 1 to obtain Col-Ncad. The preparation method of Col-BSA is the same as that of Col-Ncad.

After culturing the neural stem cells into spheres for 7 days, the resultant was digested into single cells, the cells were inoculated on the surface of Col-Ncad at a density of 5×10⁵/mL, and subjected to FDA staining after standing at 37°C for 2 hours, with BSA-crosslinked LOCS (Col-BSA) of Comparative Example 1 and Col without crosslinking any substance as controls. The statistical results of the FDA staining experiment showed that the number of neural stem cells adhered to the surface of LOCS-Ncad was significantly more than that of the controls (B1 to B4 in Figure 2).

Cell migration experiment was performed according to the schematic diagram shown in C of Figure 2, neural stem cells were obliquely inoculated on the side of crosslinked BSA, observation was performed at different time points after horizontal placement, and it is found that more neural stem cells migrate to the Col-Ncad side (D1 to D6 in Figure 2).

After 7 days' differentiation of neural stem cells on the surface of Col-Ncad, immunofluorescence staining was used to determine the proportion of Tuj-1 (a marker of neuron) and GFAP (an astrocyte marker) positive cells. Further, the changes in the expression levels of Tuj-1 and GFAP in neural stem cells on the surface of Col-Ncad were detected using qPCR. The results are shown in Fig. 3, and the results show that the proportion of Tuj-1 positive cells on the surface of Col-Ncad is significantly higher than that of Col-BSA and uncrosslinked Col, while the proportion of GFAP positive cells is significantly lower than that of Col-BSA and uncrosslinked Col, proving that Col-Ncad can efficiently promote the differentiation of neural stem cells into neurons.

### Experimental Example 4: Migration of neural stem cells and their differentiation into neurons in vivo promoted by LOCS-Ncad

LOCS-Ncad prepared in Example 1 was transplanted into a rat model of complete transection spinal cord injury, and the effects of LOCS-Ncad on promoting the migration of neural stem cells and their differentiation into neurons were analyzed *in vivo.*

Sample slices were taken 10 days after injury for immunofluorescence staining, and the results showed that as compared with the LOCS transplantation group and control group without any treatment, more Nestin⁺ neural stem cells migrated to the center of the injury region (A1 to A13 of Figure 4), and more newborn neurons appeared in the central region (B1 to B13 of Figure 4) at the same time. The result of staining at 30 days post-injury showed that as compared with the LOCS transplantation group and the control group without any treatment, more newborn neurons appeared in the center of the injury region (C1 to C13 in Figure 4), and LOCS-Ncad significantly inhibited the deposition of chondroitin sulfate proteoglycan (CSPG) (Figure 5).

### Experimental Example 5: Recovery of electrophysiological and motor functions in rats with complete transected spinal cord injury promoted by LOCS-Ncad

LOCS-Ncad prepared in Example 1 was transplanted into the rat model of complete transected spinal cord injury to analyze the effects of LOCS-Ncad transplantation on the recovery of electrophysiological and motor functions in rats with complete transected spinal cord injury.

The electrophysiological test of cortical evoked potential was performed 30 days after injury. The results showed that as compared with the LOCS transplantation group and control group without any treatment, the latency of the LOCS-Ncad transplantation group was significantly reduced (A and B in Figure 6). BBB score and oblique plate test were conducted once a week, and the test data of the LOCS-Ncad transplantation group was also significantly higher than that of the control groups (C and D in Figure 6).

The LOCS-Ncad prepared in Example 2 was also subjected to the experimental verification of Experimental Examples 2 to 5, and the results showed that the LOCS-Ncad prepared in Example 2 can achieve substantially the same performance and effect as the LOCS-Ncad prepared in Example 1.

Although the general description, specific embodiments and experiments have been used to describe the present invention in detail above, some modifications or improvements can be made on the basis of the present invention, which is obvious to a person skilled in the art. Therefore, all such modifications or improvements made without departing from the scope of the present invention as defined in the appended claims, belong to the scope of the present invention.

### Industrial applicability

The present invention provides a tissue engineering material for nerve injury repair, a preparation method and an application thereof. The tissue engineering material for nerve injury repair provided by the present invention is an N-cadherin covalently crosslinked linear ordered collagen scaffold. The tissue engineering material prepared by crosslinking N-cadherin with a linear ordered collagen scaffold can efficiently induce the migration of the neural stem cells towards an injury region, so that the neural stem cells are enriched in the injury region, and can effectively inhibit the deposition of inhibitory factors such as chondroitin sulfate proteoglycan, promote the differentiation of the neural stem cells into neurons, and further promote the recovery of electrophysiological and motor functions. At the same time, the N-cadherin covalently crosslinked linear ordered collagen scaffold also has a stable and ordered topology structure and excellent mechanical properties, can be used to repair nerve injuries such as spinal cord injury, and has good economic value and application prospect.

## Claims

1. A tissue engineering material for nerve injury repair, wherein, the tissue engineering material for nerve injury repair is a N-cadherin covalently crosslinked biodegradable material, wherein the biodegradable material is collagen; preferably a linear ordered collagen scaffold.

2. The tissue engineering material of claim 1, wherein
a ratio of the linear ordered collagen scaffold to the N-cadherin is one linear ordered collagen scaffold having a diameter of 0.25mm and a length of 4cm: (1-6) µg of N-cadherin.

3. The tissue engineering material according to claim 1 or 2, wherein the N-cadherin is covalently crosslinked with the linear ordered collagen scaffold;
preferably, the crosslinking agent for covalent crosslinking is Traut's reagent and Sulfo-SMCC.

4. A preparation method for a tissue engineering material for nerve injury repair, **characterized by** comprising: crosslinking N-cadherin with a biodegradable material covalently to obtain a N-cadherin crosslinked biodegradable material wherein the biodegradable material is collagen; more preferably a linear ordered collagen scaffold.

5. The preparation method according to claim 4, wherein a ratio of the linear ordered collagen scaffold to N-cadherin is one linear ordered collagen scaffold having 0.25mm in diameter and 4cm in length : (1-6) µg of N-cadherin;
preferably, a ratio of the linear ordered collagen scaffold to N-cadherin is one linear ordered collagen scaffold having a diameter of 0.25mm and a length of 4cm: (2.5-5) µg of N-cadherin.

6. The preparation method according to claim 4 or 5, **characterized by** comprising the following steps:
(1) treating the linear ordered collagen scaffold with Traut's reagent to obtain a linear ordered collagen scaffold modified by Traut's reagent; and
(2) treating the linear ordered collagen scaffold modified by Traut's reagent with Sulfo-SMCC and N-cadherin.

7. The preparation method according to claim 6, wherein a ratio of the linear ordered collagen scaffold to the Traut's reagent is one linear ordered collagen scaffold having a diameter of 0.25mm and a length of 4cm: (0.05-0.15) mg of Traut's reagent;
a molar ratio of the N-cadherin to the Sulfo-SMCC is 1 : (60-100);
preferably, a concentration of the Traut's reagent is 0.2mg/mL to 5mg/mL.

8. The preparation method according to claim 6 or 7, wherein the treatment time in step (1) is 1h to 3h; and the treatment time in the step (2) is 1h to 3h;
preferably, both step (1) and step (2) are carried out under the condition of 18°C to 25°C.

9. Use of the tissue engineering material according to any one of claims 1 to 3 or use of the preparation method according to any one of claims 4 to 8 in preparing a material for nerve injury repair;
preferably, the nerve injury is a spinal cord nerve injury.

10. The tissue engineering material according to any one of claims 1 to 3 for use in a method for nerve injury repair.

11. The tissue engineering material for use according to claim 10, wherein said material promotes directed migration of neural stem cells or promotes differentiation of neural stem cells into neurons.

## Patentansprüche

1. Gewebezüchtungsmaterial zur Reparatur von Nervenläsionen, wobei das Gewebezüchtungsmaterial zur Reparatur von Nervenläsionen ein kovalent vernetztes, biologisch abbaubares N-Cadherin-Material ist, wobei das biologisch abbaubare Material Kollagen ist; vorzugsweise ein linear geordnetes Kollagengerüst.

2. Gewebezüchtungsmaterial nach Anspruch 1, wobei
ein Verhältnis des linear geordneten Kollagengerüsts zum N-Cadherin ein linear geordnetes Kollagengerüst mit einem Durchmesser von 0,25 mm und einer Länge von 4 cm ist: (1-6) µg N-Cadherin.

3. Gewebezüchtungsmaterial nach Anspruch 1 oder 2, wobei das N-Cadherin mit dem linear geordneten Kollagengerüst kovalent vernetzt ist;
wobei das Vernetzungsmittel für die kovalente Vernetzung vorzugsweise Trauts-Reagenz und Sulfo-SMCC ist.

4. Herstellungsverfahren für ein Gewebezüchtungsmaterial zur Reparatur von Nervenläsionen, **dadurch gekennzeichnet, dass** es Folgendes umfasst: kovalentes Vernetzen von N-Cadherin mit einem biologisch abbaubaren Material, um ein mit N-Cadherin vernetztes biologisch abbaubares Material zu erhalten, wobei das biologisch abbaubare Material Kollagen ist; bevorzugter ein linear geordnetes Kollagengerüst.

5. Herstellungsverfahren nach Anspruch 4, wobei ein Verhältnis des linear geordneten Kollagengerüsts zum N-Cadherin ein linear geordnetes Kollagengerüst mit einem Durchmesser von 0,25 mm und einer Länge von 4 cm ist: (1-6) µg N-Cadherin;
wobei ein Verhältnis des linear geordneten Kollagengerüsts zum N-Cadherin vorzugsweise ein linear geordnetes Kollagengerüst mit einem Durchmesser von 0,25 mm und einer Länge von 4 cm ist: (2,5-5) µg N-Cadherin.

6. Herstellungsverfahren nach Anspruch 4 oder 5, **dadurch gekennzeichnet, dass** es die folgenden Schritte umfasst:
(1) Behandeln des linear geordneten Kollagengerüsts mit Trauts-Reagenz, um ein durch Trauts-Reagenz modifiziertes linear geordnetes Kollagengerüst zu erhalten; und
(2) Behandeln des durch Trauts-Reagenz modifizierten linear geordneten Kollagengerüsts mit Sulfo-SMCC und N-Cadherin.

7. Herstellungsverfahren nach Anspruch 6, wobei ein Verhältnis des linear geordneten Kollagengerüsts zum Trauts-Reagenz ein linear geordnetes Kollagengerüst mit einem Durchmesser von 0,25 mm und einer Länge von 4 cm ist: (0,05-0,15) mg Trauts-Reagenz;
wobei ein molares Verhältnis des N-Cadherin zu Sulfo-SMCC 1 ist: (60-100);
wobei eine Konzentration des Trauts-Reagenzes vorzugsweise 0,2 mg/ml bis 5 mg/ml ist.

8. Herstellungsverfahren nach Anspruch 6 oder 7, wobei die Behandlungszeit in Schritt (1) 1 Std. bis 3 Stdn. beträgt; und die Behandlungszeit in Schritt (2) 1 Std. bis 3 Stdn. beträgt;
wobei sowohl Schritt (1) als auch Schritt (2) vorzugsweise bei einer Temperatur von 18 °C bis 25 °C ausgeführt werden.

9. Verwendung des Gewebezüchtungsmaterials nach einem der Ansprüche 1 bis 3 oder Verwendung des Herstellungsverfahrens nach einem der Ansprüche 4 bis 8 bei der Herstellung eines Materials zur Reparatur von Nervenläsionen;
wobei es sich bei der Nervenläsion vorzugsweise um eine Läsion des Rückenmarksnervs handelt.

10. Gewebezüchtungsmaterial nach einem der Ansprüche 1 bis 3 zur Verwendung in einem Verfahren zur Reparatur von Nervenläsionen.

11. Gewebezüchtungsmaterial zur Verwendung nach Anspruch 10, wobei das besagte Material die gerichtete Migration von neuralen Stammzellen fördert oder die Differenzierung von neuralen Stammzellen in Neuronen fördert.

## Revendications

1. Matériau d'ingénierie tissulaire pour la réparation de lésions nerveuses, où le matériau d'ingénierie tissulaire pour la réparation de lésions nerveuses est un matériau biodégradable réticulé de manière covalente à la N-cadhérine, où le matériau biodégradable est du collagène, de préférence un échafaudage de collagène ordonné de manière linéaire.

2. Matériau d'ingénierie tissulaire selon la revendication 1, où un rapport entre l'échafaudage de collagène ordonné de manière linéaire et la N-cadhérine est un échafaudage de collagène ordonné de manière linéaire ayant un diamètre de 0,25 mm et une longueur de 4 cm : (1 à 6) µg de N-cadhérine.

3. Matériau d'ingénierie tissulaire selon la revendication 1 ou 2, où la N-cadhérine est réticulée de manière covalente avec l'échafaudage de collagène ordonné de manière linéaire.
de préférence, l'agent de réticulation pour une réticulation covalente est le réactif de Traut et le sulfo-SMCC.

4. Procédé de préparation d'un matériau d'ingénierie tissulaire pour la réparation de lésions nerveuses, **caractérisé en ce qu'**il comprend : la réticulation de manière covalente de la N-cadhérine avec un matériau biodégradable pour obtenir un matériau biodégradable réticulé à la N-cadhérine où le matériau biodégradable est du collagène ; plus préférablement, un échafaudage de collagène ordonné de manière linéaire.

5. Procédé de préparation selon la revendication 4, où un rapport entre l'échafaudage de collagène ordonné de manière linéaire et la N-cadhérine est un échafaudage de collagène ordonné de manière linéaire ayant 0,25 mm de diamètre et 4cm de longueur : (1 à 6) µg de N-cadhérine ;
de préférence, un rapport entre l'échafaudage de collagène ordonné de manière linéaire et la N-cadhérine est un échafaudage de collagène ordonné de manière linéaire ayant un diamètre de 0,25 mm et une longueur de 4 cm : (2,5 à 5) µg de N-cadhérine.

6. Procédé de préparation selon la revendication 4 ou 5, **caractérisé en ce qu'**il comprend les étapes suivantes :
(1) traitement de l'échafaudage de collagène ordonné de manière linéaire avec le réactif de Traut pour obtenir un échafaudage de collagène ordonné de manière linéaire modifié par le réactif de Traut ; et
(2) traitement de l'échafaudage de collagène ordonné de manière linéaire modifié par le réactif de Traut avec du sulfo-SMCC et de la N-cadhérine.

7. Procédé de préparation selon la revendication 6, où un rapport entre l'échafaudage de collagène ordonné de manière linéaire et le réactif de Traut est un échafaudage de collagène ordonné de manière linéaire ayant un diamètre de 0,25 mm et une longueur de 4 cm : (0,05 à 0,15) mg de réactif de Traut ;
le rapport molaire entre la N-cadhérine et le sulfo-SMCC est de 1 : (60 à 100) ;
de préférence, la concentration du réactif de Traut est de 0,2 mg/ml à 5 mg/ml.

8. Procédé de préparation selon la revendication 6 ou 7, où la durée de traitement à l'étape (1) est de 1h à 3h ; et la durée de traitement à l'étape (2) est de 1h à 3h ;
de préférence, l'étape (1) et l'étape (2) sont toutes deux effectuées dans des conditions allant de 18°C à 25°C.

9. Utilisation du matériau d'ingénierie tissulaire selon l'une quelconque des revendications 1 à 3 ou utilisation du procédé de préparation selon l'une quelconque des revendications 4 à 8 dans la préparation d'un matériau pour la réparation de lésions nerveuses ;
de préférence, la lésion nerveuse est une lésion nerveuse de la moelle épinière.

10. Matériau d'ingénierie tissulaire selon l'une quelconque des revendications 1 à 3 pour l'utilisation dans un procédé de réparation de lésions nerveuses.

11. Matériau d'ingénierie tissulaire pour l'utilisation selon la revendication 10, où ledit matériau favorise la migration dirigée de cellules souches neurales ou favorise la différenciation de cellules souches neurales en neurones.
